# EUROPEAN PATENT SPECIFICATION

(11) **EP 4 115 933 B1**
(45) Date of publication and mention of the grant of the patent: **29.07.2026**
(21) Application number: 21763772.7
(22) Date of filing: 05.03.2021
(51) Int. Cl.: A61M 25/00

(54) **CATHETER**
KATHETER
CATHÉTER

(30) Priority: 05.03.2020 JP 2020038156
(43) Date of publication of application: 11.01.2023
(73) Proprietor: TERUMO Kabushiki Kaisha, Tokyo 151-0072 (JP)
(72) Inventor: MOTOSE, Yuji, Ashigarakami-gun, Kanagawa 259-0151 (JP); SHIMADA, Daisuke, Fujinomiya-shi, Shizuoka 418-0015 (JP)
(74) Representative: Casalonga
(86) International application number: PCT/JP2021/008615
(87) International publication number: WO 2021/177434

(56) References cited:
- EP-A1- 0 109 657
- EP-A2- 1 466 644
- WO-A1-2014/103599
- WO-A1-2018/207758
- DE-A1- 3 045 453
- DE-A1- 3 045 453
- US-A- 3 720 210

## Description

### Technical Field

This invention relates to a catheter.

### Background Art

In general, a catheter includes a shaft having a lumen communicating from a distal end to a proximal end, and a hub disposed at the proximal end of the shaft and communicating with the lumen in order to be connected to a syringe or the like.

As a method for fixing the proximal end of the shaft to the hub, an insert molding method, an adhesion method using an adhesive, a fusion method using a laser, and the like are known.

### Citation List

### Patent Literature

PTL 1: JP-A-H10-180802
PTL 2: JP-UM-B-S63-17486
PTL 3: WO2016/092208

Documents US 3 720 210 A and EP 1 466 644 A2 discloses an indwelling catheter.
Document DE 30 45 453 Al discloses a disposable cannula and method and device for producing disposable cannula.
Document EP 0 109 657 Al discloses an improved catheter assembly.

### Summary of Invention

### Technical Problem

In an insert molding method disclosed in PTL 1, the shaft is disposed in an injection mold, a part of the shaft is pressed by a fixing pin, and a resin for the hub is injection-molded at a high temperature and a high pressure. Therefore, unlike a metal needle or the like, when a resin catheter is molded, deformation of the shaft caused by the fixing pin or displacement of the shaft in a long axis direction may occur.

In a case where an adhesive is used as in PTL 2, when a gap between an outer diameter of the shaft and a lumen of a shaft accommodation portion of the hub is too small, the adhesive does not flow around, and a gap remains between the hub and the shaft, so that a contrast agent may leak at the time of injection of the contrast agent. In reverse, when the gap is too large, the adhesive may flow into the lumen and the lumen may be narrowed.

In particular, in a support catheter that supports a guidewire in a blood vessel, a difference between an inner diameter of a shaft and an outer diameter of the guidewire passing through a lumen of the shaft is small, so that it is difficult to insert the guidewire into the lumen when the lumen is narrow.

In addition, when the adhesive is used, protective tools such as goggles and gloves are required, which imposes a burden on a worker who wears the protection tools.

In addition, when the shaft and the hub are fused by an infrared laser in PTL 3, since content of a pigment is extremely high at 10 wt% or more and two or more kinds of pigments are used, a color of the shaft cannot be freely selected.

### Solution to Problem

This invention to be described below achieves the above object.

(1) A catheter according to this invention includes: a shaft having a lumen communicating from a distal end to a proximal end and a shaft outer surface extending along a shaft long axis; and a hub attached to the proximal end of the shaft. The hub has a hub distal opening on a distal side, a shaft accommodation portion holding the shaft, and a hub proximal opening, the shaft accommodation portion has an adjacent surface, formed at a proximal end thereof, adjacent to a shaft proximal surface of the shaft, the adjacent surface has a hole communicating with the hub proximal opening and close to the shaft proximal opening, a fusion surface is formed by directly fusion-bonding a proximal side of the shaft outer surface on the proximal side and the hub along the shaft long axis, an inner diameter of the hub distal opening is larger than a diameter of the adjacent surface and the shaft accommodation portion has a smallest inner diameter at the fusion surface, wherein a proximal gap is formed between a shaft proximal end vicinity outer surface and the shaft accommodation portion in accordance with a gap which is formed between the shaft proximal surface and the adjacent surface.
(2) In the catheter according to (1), the smallest inner diameter of the shaft accommodation portion at the fusion surface may be smaller than an outer diameter of the shaft proximal surface.

### Advantageous Effect of Invention

In the catheter according to this invention, the shaft outer surface and the hub are directly fusion-bonded without an adhesive interposed between the shaft and the hub, and the inner diameter of the fusion surface is the smallest between a distal opening of the shaft accommodation portion and the adjacent surface on the proximal side, so that the shaft is firmly coupled to the hub, and the shaft is prevented from coming off from the hub when a high pressure is applied at the time of injection of a contrast agent or when the shaft is pulled out from a body.

Further, a manufacturing cost can be reduced since a positional deviation defect at the time of insert molding is less likely to occur and a large number of protective tools and exhaust ducts for protecting a worker in order to achieve safety of the worker and increase production are not required due to that it is not necessary to use an adhesive for the coupling between the hub and the shaft, that is, conflicting problems that are a reduction in manufacturing cost and a reduction in safety cost can be solved at the same time.

### Brief Description of Drawings

[FIG. 1] FIG. 1 is a plan view showing a partial cross-section of a catheter according to this invention.
[FIG. 2] FIG. 2 is an enlarged vertical cross-sectional view taken along a long axis X of a hub and a shaft proximal portion.

### Description of Embodiments

Hereinafter, a preferred embodiment according to this invention will be described in detail with reference to the accompanying drawings. Dimensional ratios in the drawings are exaggerated for convenience of description and may differ from actual ratios.

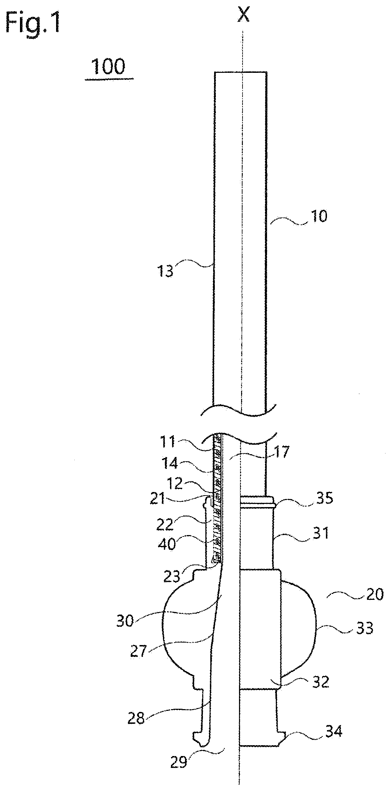

A catheter 100 according to this invention includes a shaft 10 and a hub 20 as shown in FIG. 1. In addition to a catheter supporting a guidewire, the catheter may be a guiding catheter, an angiographic catheter, or a microcatheter, or may be a balloon catheter or an imaging catheter having a lumen for inflating.

In addition, the catheter may be of an over-the-wire (OTW) type in which the shaft and the hub communicate from a distal end to a proximal end, or may be of a type having a lumen in which a guidewire lumen at the distal end of the catheter is opened in the middle of the shaft and communicates from a balloon at the distal portion to the hub at the proximal end of the catheter, such as a rapid exchange (RX) balloon catheter.

The shaft 10 has a lumen 17 that communicates the shaft 10 from a distal end to a proximal end, and an outer surface 13 extending from the distal end to the proximal end.

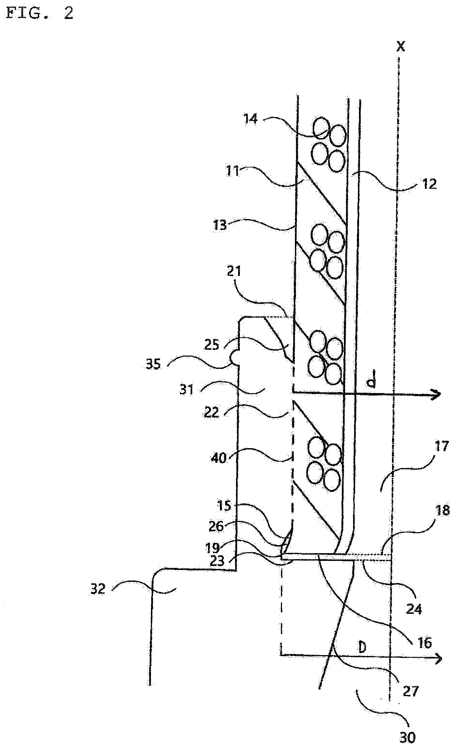

As shown in FIG.2, the shaft 10 has, on a proximal side thereof, a shaft proximal surface 16 formed by cutting the proximal end of the shaft 10 perpendicularly to a shaft long axis X, and has a shaft proximal opening 18 which is a proximal end of the lumen.

The shaft outer surface 13 has a shaft proximal end vicinity outer surface 15 extending from the proximal end toward the distal end of the shaft, and a fusion surface 40 fusion-bonded with the hub, and the shaft outer surface 13 is accommodated in a shaft accommodation portion 22 from a distal end portion of the fusion surface 40 to the shaft proximal opening 18.

As shown in FIGS. 1 and 2, the hub 20 has a hub lumen 30 that communicates the hub 20 from a proximal end to a distal end of the hub on an inner surface of the hub 20. The hub lumen 30 has a hub distal opening 21 and the shaft accommodation portion 22 in order from a distal side, and further has, at a proximal end of the shaft accommodation portion 22, an adjacent surface 23 adjacent to the shaft proximal surface 16. At a center of the adjacent surface 23, a hole 24 is formed at a position facing the shaft proximal opening 18 of the lumen 17. An inner diameter of the hub distal opening 21 is larger than a diameter of the adjacent surface 23. Therefore, the shaft 10 can be easily inserted into the shaft accommodation portion 22.

The hole 24 has a substantially circular shape around a long axis, or a substantially cylindrical shape or a substantially circular truncated cone shape having a length in a long axis direction, is coaxial with the shaft accommodation portion 22, and preferably is coaxial with the lumen 17, and it is still preferable that an inner diameter of the shaft and an inner diameter of the hole 24 are the same.

A proximal end of the hole 24 communicates with a tapered portion 27 that expands toward a proximal direction, a proximal end of the tapered portion 27 communicates with a hub proximal opening 29, and a part of the tapered portion 27 may be a luer tapered portion 28 that can be interlocked with a syringe (not shown).

Accordingly, a guidewire or a treatment catheter inserted from the hub proximal opening 29 can smoothly pass through the hub lumen 30, protrude from the catheter distal end through the shaft lumen 17, and easily reach a target position such as a lesion area.

An outer side of the hub 20 includes, in order from the distal side, a hub distal portion 31 including the shaft accommodation portion 22, a body portion 32 that can be gripped at the time of surgery and on which a display of a catheter size can be described, a wing 33, and a hub connector portion 34 that has a screw thread projection or the like that engages with a lock type syringe or the like and has the hub proximal opening 29.

In addition, the hub distal portion 31 may be provided with a projection portion 35 fitted into a hole portion or a recess portion of an anti-kink protector (not shown).

Next, a method for fusing the shaft 10 and the hub 20 will be described. The proximal end of the shaft 10 is inserted into the shaft accommodation portion 22 and the shaft proximal surface 16 is brought close to the adjacent surface 23, but the shaft proximal surface 16 may attach to the adjacent surface 23, or an outer edge of the shaft proximal surface 16 may abut against an inner surface of the shaft accommodation portion 22 on a distal side of the adjacent surface 23 with a gap between the shaft proximal surface 16 and the adjacent surface 23.

When a mandrel (not shown) is inserted into the lumen 17 of the shaft and the shaft outer surface 13 and the shaft accommodation portion 22 of the hub 20 are heated, the shaft outer surface 13 and the shaft accommodation portion 22 are melted to form the fusion surface 40.

A heating method is not particularly limited, and examples of the heating method include a method of emitting electromagnetic waves having a wavelength that can pass through the hub 20 but cannot pass through the shaft outer surface 13.

Since electromagnetic waves cannot pass through the shaft outer surface 13, first, the shaft outer surface 13 is heated and melted, the heat thereof is transmitted to the shaft accommodation portion 22 to melt the shaft accommodation portion 22, and thereby forming the fusion surface 40.

The electromagnetic waves include infrared rays in addition to heat, microwaves, and visible light. The infrared rays include near-infrared rays having a wavelength of about 0.7 µm to 2.5 µm, mid-infrared rays having a wavelength of about 2.5 µm to 4 µm, and far-infrared rays having a wavelength of about 4 µm to 1000 µm, and the electromagnetic waves may include one or more kinds of the near-infrared rays, the mid-infrared rays, and the far-infrared rays, and may include the visible light or the microwaves.

A method for emitting the electromagnetic waves is not particularly limited, and a fiber laser, a semiconductor solid-state laser such as a YAG laser using neodymium, or the like may be used.

The expression that "the electromagnetic waves can pass through" means that an object appears to be transparent to naked eyes with respect to the visible light, and that a sheet having a thickness of 0.4 mm to 0.5 mm and produced by melting and pressing resin pellets has a transmittance (hereinafter, referred to as "transmittance") of 80% or more, preferably 85% or more with respect to a wave having a specific wavelength when being measured using a spectroscopic analyzer, for example, a Fourier transform infrared and near-infrared spectroscopic analyzer. Therefore, since the expression that "the electromagnetic waves can pass through" is not limited to a case that an object is transparent with respect to the visible light, a case that an object is transparent with respect to a wave having a specific wavelength even if the object appears to be colored or opaque to naked eyes is included.

In addition, the expression that "the electromagnetic waves cannot pass through" means that an object appears to be opaque or colored to naked eyes with respect to the visible light, and that an object has a transmittance of less than 80%, preferably less than 10%, still preferably less than 1%. Therefore, since the expression that "the electromagnetic waves cannot pass through" is not limited to a case that an object is opaque or colored with respect to the visible light, a case that an object is opaque with respect to a wave having a specific wavelength or absorbs the wave even if the object appears to be transparent to naked eyes is included.

The shaft 10 of the catheter 100 shown in FIG. 2 has an outer layer 11 and an inner layer 12, and a reinforcing wire 14 obtained by weaving a metal wire or the like is formed between the outer layer 11 and the inner layer 12. In the outer layer 11, a pigment that does not transmit or absorbs heat or the electromagnetic waves may be mixed in an amount of 0.01 wt% or more and less than 10 wt%, preferably 0.05 wt% or more and 5 wt% or less, and still preferably 0.1 wt% or more and 1 wt% or less of a total of a resin.

Alternatively, a resin may be one that does not contain a pigment, a contrast agent, or the like and has a low transmittance with respect to a wave having a specific wavelength, or the resin may be replaced with a pigment or mixed with a radiopaque metal with a pigment.

The pigment is not particularly limited as long as the pigment is a pigment that develops white, black, blue, red, or yellow, or a mixture thereof, and a black pigment, for example, carbon black is preferable as the pigment that easily absorbs the electromagnetic waves. The radiopaque materials are, for example, a compound of gold, bismuth, or tungsten, and a powder radiopaque materials are more preferable.

Alternatively, the outer layer may be one that has a low transmittance with respect to a wave having a specific wavelength even if the outer layer is transparent to naked eyes.

Examples of the resin of the outer layer include, in addition to a polyamide resin, a polyester resin, a polyolefin resin, and a polyurethane resin, a polyamide elastomer, a polyester elastomer, a polyurethane elastomer, or a mixture of one or more of these resins or elastomers, or a mixture of the resins or elastomers having different hardness. These elastomers having different hardness may be arranged such that the outer layer 11 is flexible from the proximal end toward the distal end.

A resin of the inner layer may be the same as that of the outer layer, or may be a resin different from that of the outer layer, or may be a polytetrafluoroethylene resin in order to improve sliding property of the inner surface.

The hub 20 is not particularly limited as long as the hub 20 is made of a thermoplastic resin that can be injection-molded, preferably, the thermoplastic resin can easily transmits the heat or the electromagnetic waves, and specific examples of the thermoplastic resin include a polyolefin resin, a polyamide resin, a polycarbonate resin, and a polyester resin.

For example, when the shaft 10 and the hub 20 are fused by being irradiated with an infrared laser, electromagnetic waves transmitted through the hub 20, which is transparent with respect to a wave having a wavelength of the irradiated infrared laser, are absorbed by the opaque resin, pigment, or the like of the outer layer 11 of the shaft to mainly generate heat, the resin of the outer layer 11 of the shaft is melted, the heat is transferred to the shaft accommodation portion 22 of the hub 20, and at least a part of an inner surface of the shaft accommodation portion 22 is melted. When the inner surface of the shaft accommodation portion 22 is melted, an inner diameter of the shaft accommodation portion 22 is reduced, the shaft accommodation portion 22 is brought into contact with the shaft outer surface 13, and the fusion surface 40 is formed.

Accordingly, since the inner diameter of the shaft accommodation portion 22 is the smallest at the fusion surface 40, the shaft 10 is less likely to come off from the hub 20.

The inner diameter of the shaft accommodation portion 22 at the fusion surface 40 being smaller than an inner diameter of the adjacent surface 23 means that, for example, the inner diameter at the fusion surface 40 is smaller than the inner diameter of the adjacent surface 23 when the inner diameter at the fusion surface 40 and the inner diameter of the adjacent surface 23 are compared in a cross-section perpendicular to the shaft long axis.

A difference between the inner diameter of the shaft accommodation portion 22 at the fusion surface 40 and the inner diameter of the adjacent surface 23 is 0 mm or more and 0.5 mm or less, preferably more than 0 mm and 0.2 mm or less. Since the shaft proximal surface 16 can be inserted to the shaft accommodation portion 22 only on a distal side of the adjacent surface 23 when the difference is less than 0 mm, a gap is generated between the shaft proximal surface 16 and the adjacent surface 23.

When the difference between the inner diameter of the shaft accommodation portion 22 at the fusion surface 40 and the inner diameter of the adjacent surface 23 is more than 0.5 mm, the fusion surface 40 between the shaft 10 and the hub 20 may be not sufficiently formed and tensile strength may be reduced.

In addition, a distal gap 25 may be formed between the hub distal opening 21 and the fusion surface 40 on the distal side. The distal gap 25 may be defined as a difference between an outer diameter of the shaft and the inner diameter of the hub accommodation portion 22 that changes along the shaft long axis X, or may be defined as a difference between the outer diameter of the shaft and an inner diameter of the distal gap 25 on a proximal side of the hub distal opening 21.

The difference between the outer diameter of the shaft and the inner diameter of the shaft accommodation portion 22 at the distal gap 25 may be a gap through which the shaft 10 can be inserted into the shaft accommodation portion 22, and is more than 0 mm and 0.2 mm or less.

An inner diameter of the shaft proximal opening 18 may be set larger than the inner diameter of the hole 24 to form a flare portion 19, and a mandrel may be inserted into the lumen 17 to enlarge a diameter of the shaft proximal surface 16 so as not to hook a guidewire to the shaft proximal opening 18 when the guidewire (not shown) is inserted.

Accordingly, since the inner diameter of the shaft accommodation portion 22 at the fusion surface 40 is smaller than an outer diameter of the flare portion 19 that is the shaft proximal surface 16, the shaft 10 can be prevented from coming off from the hub 20.

In addition, a proximal gap 26 is formed between the shaft proximal end vicinity outer surface 15 and the shaft accommodation portion 22 in accordance with a gap between the shaft proximal surface 16 and the adjacent surface 23.

Processing time can be shortened by shortening the fusion surface 40.

Accordingly, the shaft 10 is prevented from being pulled out from the hub 20 when a high pressure is applied at the time of injecting, in order to observe a state of a treatment site, an X-ray contrast agent by a contrast agent injection device (not shown) connected to the luer tapered portion 28, or when the hub 20 is pulled at the time of pulling the shaft 10 inserted into the body out of the body after treatment is performed using the catheter 100.

Although the preferred embodiment according to this invention has been described above, this invention is not limited to the above embodiment, and it is needless to say that various modifications can be made without departing from the gist of this invention.

The present application is based on Japanese Patent Application No. 2020-38156, filed on March 5, 2020.

### Reference Signs List

10 shaft
11 outer layer
12 inner layer
13 shaft outer surface
14 reinforcing wire
15 shaft proximal end vicinity outer surface
16 shaft proximal surface
17 lumen
18 shaft proximal opening
19 flare portion
20 hub
21 hub distal opening
22 shaft accommodation portion
23 adjacent surface
24 hole
25 distal gap
26 proximal gap
27 tapered portion
28 luer tapered portion
29 hub proximal opening
31 hub distal portion
32 hub body portion
33 wing
34 hub connector portion
35 projection portion
40 fusion surface
X shaft long axis

## Claims

1. A catheter comprising:
a shaft (10) having a lumen (17) communicating from a distal end to a proximal end, a shaft proximal surface (16), a shaft proximal opening (18) provided in the shaft proximal surface (16) and communicating with the lumen (17), and a shaft outer surface (13) extending along a shaft long axis (X); and
a hub (20) attached to the proximal end of the shaft (10), wherein
the hub (20) has a hub distal opening (21) on a distal side, a shaft accommodation portion (22) holding the shaft (10), and a hub proximal opening (29),
the shaft accommodation portion (22) has an adjacent surface (23), formed at a proximal end thereof, adjacent to the shaft proximal surface (16) of the shaft (10),
the adjacent surface (23) has a hole (24) communicating with the hub proximal opening (29) and close to the shaft proximal opening (18),
a fusion surface (40) is formed by directly fusion-bonding a proximal side of the shaft outer surface (13) and the hub (20) along the shaft long axis (X), and
an inner diameter of the hub distal opening (21) is larger than a diameter of the adjacent surface (23) and the shaft accommodation portion (22) has a smallest inner diameter at the fusion surface (40), wherein
a proximal gap (26) is formed between a shaft proximal end vicinity outer surface (15) and the shaft accommodation portion (22) in accordance with a gap which is formed between the shaft proximal surface (16) and the adjacent surface (23).

2. The catheter according to Claim 1, wherein
the smallest inner diameter of the shaft accommodation portion (22) at the fusion surface (40) is smaller than an outer diameter of the shaft proximal surface (16).

3. The catheter according to any of Claims 1 or 2, wherein
the shaft (10) has an outer layer (11) and an inner layer (12), and a reinforcing wire (14) obtained by weaving a metal wire or the like is formed between the outer layer (11) and the inner layer (12).

## Patentansprüche

1. Katheter, umfassend:
einen Schaft (10), der ein Lumen (17), das von einem distalen Ende zu einem proximalen Ende in Verbindung steht, eine proximale Schaftoberfläche (16), eine proximale Schaftöffnung (18), die in der proximalen Schaftoberfläche (16) vorgesehen ist und mit dem Lumen (17) in Verbindung steht, und eine äußere Schaftoberfläche (13) aufweist, die sich entlang einer Schaftlängsachse (X) erstreckt; und
eine Nabe (20), die an dem proximalen Ende des Schafts (10) angebracht ist, wobei
die Nabe (20) eine distale Nabenöffnung (21) an einer distalen Seite, einen den Schaft (10) aufnehmenden Schaftaufnahmeabschnitt (22) und eine proximale Nabenöffnung (29) aufweist,
der Schaftaufnahmeabschnitt (22) eine angrenzende Oberfläche (23) aufweist, die an einem proximalen Ende davon benachbart zu der proximalen Schaftoberfläche (16) des Schaftes (10) ausgebildet ist,
die angrenzende Oberfläche (23) ein Loch (24) aufweist, das mit der proximalen Nabenöffnung (29) in Verbindung steht und nahe der proximalen Öffnung (18) des Schafts liegt,
eine Verschmelzungsfläche (40) durch direktes Schmelzverbinden einer proximalen Seite der äußeren Schaftoberfläche (13) und der Nabe (20) entlang der Schaftlängsachse (X) ausgebildet ist, und
ein Innendurchmesser der distalen Nabenöffnung (21) größer ist als ein Durchmesser der angrenzenden Oberfläche (23) und der Schaftaufnahmeabschnitt (22) an der Verschmelzungsfläche (40), wobei
ein proximaler Spalt (26) zwischen einer Außenfläche (15) in der Nähe des proximalen Endes des Schaftes und dem Schaftaufnahmeabschnitt (22) entsprechend einem Spalt gebildet ist, der zwischen der proximalen Schaftoberfläche (16) und der angrenzenden Oberfläche (23) ausgebildet ist.

2. Katheter nach Anspruch 1, wobei
der kleinste Innendurchmesser des Schaftaufnahmeabschnitts (22) an der Verschmelzungsfläche (40) kleiner ist als ein Außendurchmesser der proximalen Schaftoberfläche (16).

3. Katheter nach einem der Ansprüche 1 oder 2, wobei
der Schaft (10) eine Außenschicht (11) und eine Innenschicht (12) aufweist und ein Verstärkungsdraht (14), der durch Verflechten eines Metalldrahtes oder dergleichen hergestellt wurde, zwischen der Außenschicht (11) und der Innenschicht (12) ausgebildet ist.

## Revendications

1. Cathéter comprenant :
une tige (10) présentant une lumière (17) communiquant d'une extrémité distale à une extrémité proximale, une surface proximale (16) de tige, une ouverture proximale (18) de tige disposée dans la surface proximale (16) de tige et communiquant avec la lumière (17), et une surface extérieure (13) de tige s'étendant le long d'un axe long (X) de tige ; et
une embase (20) fixée à l'extrémité proximale de la tige (10), dans lequel
l'embase (20) présente une ouverture distale (21) d'embase sur un côté distal, une partie de réception (22) de tige maintenant la tige (10), et une ouverture proximale (29) d'embase,
la partie de réception (22) de tige présente une surface adjacente (23), formée à une extrémité proximale de celle-ci, adjacente à la surface proximale (16) de tige de la tige (10),
la surface adjacente (23) présente un orifice (24) communiquant avec l'ouverture proximale (29) d'embase et proche de l'ouverture proximale (18) de tige,
une surface de fusion (40) est formée par liaison par fusion directe d'un côté proximal de la surface extérieure (13) de tige et de l'embase (20) le long de l'axe long (X) de tige, et
un diamètre intérieur de l'ouverture distale (21) d'embase est plus grand qu'un diamètre de la surface adjacente (23) et la partie de réception (22) de tige présente un plus petit diamètre intérieur au niveau de la surface de fusion (40), dans lequel
un espace proximal (26) est formé entre une surface extérieure (15) près de l'extrémité proximale de tige et la partie de réception (22) de tige conformément à un espace qui est formé entre la surface proximale (16) de tige et la surface adjacente (23).

2. Cathéter selon la revendication 1, dans lequel
le plus petit diamètre intérieur de la partie de réception (22) de tige au niveau de la surface de fusion (40) est inférieur à un diamètre extérieur de la surface proximale (16) de tige.

3. Cathéter selon l'une quelconque des revendications 1 ou 2, dans lequel
la tige (10) présente une couche extérieure (11) et une couche intérieure (12), et un fil de renfort (14) obtenu par tissage d'un fil métallique, ou analogue, est formé entre la couche extérieure (11) et la couche intérieure (12) .
